# EUROPEAN PATENT APPLICATION

(11) **EP 1 609 465 A1**
(43) Date of publication of application: **28.12.2005**
(21) Application number: 04724177.3
(22) Date of filing: 29.03.2004
(51) Int. Cl.: A61K 9/107, A61K 47/34, A61K 47/42, A61K 31/409, A61P 27/02

(54) **OPHTHALMIC DRUG DELIVERY SYSTEM USING POLYMER MICELL**

(30) Priority: 28.03.2003 JP 2003089485; 29.05.2003 JP 2003151876; 05.09.2003 JP 2003313808
(71) Applicant: Nanocarrier Kabushiki Kaisha, Kashiwa-shi, Chiba 277-0882 (JP)
(72) Inventor: KATAOKA, Kazunori, 1650031 (JP); TAMAKI, Yasuhiro, 1140024 (JP); HARADA, Atsushi, 5918023 (JP); TASAKA, Fumitaka, 5750003 (JP)
(74) Representative: Peaucelle, Chantal
(86) International application number: PCT/JP2004/004412
(87) International publication number: WO 2004/087103

(57) **Abstract**

The current situation is such that it is difficult for drugs to reach posterior tissues of eyeball, such as choroid and retina, with the result that treating diseases in such regions is difficult. Thus, how to attain effective delivery of drugs is of importance. It has been found that effective delivery of drugs to positerior tissues of eyeball, such as choroid and retina, especially those wherein vascularization has occurred can be accomplished by systemic administration, especially intravenous administration of polymer micells having a drug incorporated therein. Further, it has been found that when a photosensitive substance is used as a drug and PDT is carried out, choroidal neo-vessels can be effectively choked so as to be useful for the treatment of age-related macular degeneration.

## Description

### Technical Field

The present invention relates to an ophthalmic drug delivery system characterized by efficiently delivering a drug to posterior tissues of eyeball such as choroid and retina, especially those wherein neovascularization has occured, by administering a polymer micelle having a drug incorporated thereinin that is useful as a therapeutic drug for an ocular disease. The drug delivery system of the invention can be effectively applied to photodynamic therapies when a photosensitive substance is used as the drug, and can be subjected to therapy for age-related macular degeneration or the like through occluding choroidal new vessels.

### Background Art

Polymer micelles are nanoparticles fundamentally formed with a hydrophilic polymer chain as a shell and a hydrophobic polymer chain as a core. Various studies have been made on the polymer micelles for drug solubilization or as a carrier for drug delivery. Examples of the studies involve: a report on a carrier for retaining a drug, the carrier being a block copolymer in which a polyalkylene oxide, polymalic acid, polyaspartic acid or the like is used as the hydrophilic polymer chain, and a hydrophobic polyamino acid, polystyrene, polymethacrylate or the like is used as the hydrophobic polymer chain (Japanese Patent No. 2777530), a report on a micelle incorporating a polyene antibiotics such as amphoterin B as a drug incorporated into the block copolymer as described above (Japanese Patent No. 3220069), reports on a process for preparing a polymer micelle incorporating a slightly-solu' drug in water (JP-A-11-335367, JP-A-2001-226294, JP-A-2003-26812), and a report on a method of stabilization of a polyion complex micelle having a core-shell structure formed with a block copolymer having a hydrophilic polymer chain such as a polyethylene glycol and a charged polymer chain such as a polyamine or a polycarboxylic acid, and a polymer electrolyte such as a polypeptide or a polypseudo peptide (JP-A-2001-146556).

Also, there is a report to improve permeability of a drug in cornea by preparing a solution containing the drug loaded into a polymer micelle, and administering eye drops of the same (United States Patent Publication No. 2002/0064513), and a report intending DDS to anterior chamber (JP-A-10-510293).

However, these reports do not describe possibility of delivery of the drug to posterior tissues of eyeball such as choroid or retina, especially those wherein neovascularization has occurred.

In addition, it was reported that a method of therapy for age-related macular degeneration (AMD) through administration of a photosensitive substance by intravenous injection or the like, followed by irradiating a laser beam to the diseased part to allow the occlusion of new choroidal vessels, thus the generated radical, i.e., a photodynamic therapy (PDT), becomes useful in the treatment of age-related macular degeneration (VISION TIMES Vol. 8, No. 2, pp. 7-9 (2001)). As the photosensitive substance which may be used in this therapy for AMD, porphyrin derivatives have been well known, and VISION TIMES Vol. 8, No. 2, pp. 7-9 (2001) presents verteporfin, SnET2, ATX-S10, MV6401 and the like. Among them, verteporfin has been already used in practice for AMD therapy in USA.

Moreover, as a porphyrin derivative which may be used in the PDT for cancer and the like, temoporfin (Scrip Daily Online, January, 28, 2001, S00716156), talaporfin (Scrip Daily Online, January, 15, 2001, S00693630) and the like have been known.

Further, in connection with porphyrin derivatives which may be used in the PDT, a number of patent applications are found. For example, USP No. 5707986, WO2001/82860, JP11-502520T, JP2001-514658T, WO2002/96365 and the like may be exemplified.

However, any of the above documents does not refer to a technique of using a polymer micelle.

On the other hand, a technique of using a PDT in which a photosensitive substance is incorporated into a polymer micelle was reported (Japanese Patent No. 3422481). Dendrimer-type porphyrin is described therein as a porphyrin derivative that is a photosensitive substance, which can be suitably used in a PDT for cancer and the like. However, Japanese Patent No. 3422481 does not describe ophthalmic application, and possibility of delivery of the photosensitive substance to a posterior tissue of eyeball has been unknown. Still more, applicability to AMD could not be expected.

### Disclosure of the Invention

In a therapy for ocular diseases, a drug is administered in eye drops, in general. However, the drug hardly reaches posterior tissues of eyeball such as choroid and retina in administration of eye drops, therefore, therapy for diseases in such sites has been difficult under the current situations. Thus, there has been a problem on how a drug is efficiently delivered to a posterior tissue of eyeball.

Furthermore, many diseases of posterior segment of eyeball are intractable. Among them, AMD is caused by choroidal neovascularization, and is referred to as a major cause of blindness. Particularly, exudative AMD is a disease that causes severe visual loss, and therapy thereof is very difficult. Recently, as a therapeutic method for this exudative AMD, a PDT using a photosensitive substance was developed. Typical photosensitive substance therefor is a porphin derivative, and as described in the section of Background Art, various porphin derivatives have been developed. Therapy for AMD is intended by this PDT through administration of a photosensitive substance by intravenous injection or the like, followed by irradiating a laser beam to the accumulated photosensitive substance on the diseased part to generate a radical (singlet oxygen) thereby allowing for occlusion of new vessels by the singlet oxygen. Because irradiation of a laser beam may affect ocular tissues, continuous repetition of irradiation is difficult in the prior art. Therefore, the therapy has been merely used for preventing the onset of blindness for a certain period of time.

Accordingly, as a result of elaborate investigation taking into account of polymer micelles that have been studied as a carrier for drug delivery, it was found that a drug can be efficiently delivered to a posterior tissue of eyeball such as choroid or retina, especially those wherein neovascularization has occurred, by intravenous administration of a polymer micelle incorporating the drug.

Moreover, it was found that use of the polymer micelle enables, in addition to stabilization of the photosensitive substance, efficient accumulation in choroidal new vessels, thereby permitting efficacious occlusion of the new vessels even with a low dose of laser. Still further, it was found that a photosensitive substance can be solubilized in water by using the polymer micelle thereby leading to ease in administration.

The present invention relates to an ophthalmic drug delivery system characterized by efficiently delivering a drug to a posterior tissue of eyeball by administering a polymer micelle incorporating the drug.

The polymer micelle used in the invention is a nanoparticle fundamentally formed with a hydrophilic polymer chain as a shell and a hydrophobic polymer chain as a core. Particle size of the micelle is 10 nm or greater and 100 nm or less, and is preferably approximately several tens nm. As the polymer micelle, known micelles, e.g., any one of the micelles described in the foregoing first to sixth Japanese Patents and Publications of Patent Application may be used.

Examples of the hydrophilic polymer chain which may be used include a polyalkylene oxide such as polyoxyethylene, polyethylene glycol, polymalic acid, polyaspartic acid and the like. Examples of the hydrophobic polymer chain which may be used include polylactone, hydrophobic polyamino acid, polystyrene, polymethacrylate ester and the like. A block copolymer is formed between the hydrophilic polymer chain and the hydrophobic polymer chain. As the core, an anionic or cationic charged polymer chain such as a polypeptide (such as polyaspartic acid), a polyamine or a polycarboxylic acid may be used. Also, a polyion complex of core-shell type having a core comprising the aforementioned charged polymer chain and a polymer electrolyte such as a polypeptide (such as polylysine) or a polypseudo peptide may be suitably used.

A process having a drug incorpotrated may be also carried out according to any known method, and examples of the method which might be used include a physical incorporation method of a drug into a micelle, a method to allow for covalent binding with the polymer that forms a micelle, and a method to allow for ionic bonding with the drug using a charged polymer chain when the drug is ionic.

The invention is characterized by efficiently delivering a drug to a posterior tissue of eyeball such as choroid or retina,especially those wherein neovascularization has occured. The drug may be of any kind without limitation, but may be any one used in therapy for diseases of posterior tissues of eyeball such as choroid and retina.

Method of administration of the polymer micelle incorporating a drug is not particularly limited, but intravenous injection is preferred.

Advantage of the invention will be described in detail in the section of Example, in which intraocular transfer of a polymer micelle was investigated when a fluorescence-lebelled polymer micelle was systemically administered (intravenous injection) to a rat choroidal neovascular (CNV) model. Consequently, accumulation of intense fluorescence agreeing with choriocapillaris vascular lamina and CNV was found one hour after the administration. Twenty four hours after the administration, fluorescence in normal choriocapillaris vascular lamina was attenuated, however, accumulation of intense fluorescence agreeing with CNV remained. In other words, it was revealed that the polymer micelle exhibits high accumulating capability for CNV, and is effective as a DDS for CNV.

In addition, when a photosensitive substance is used as a drug, it can be effectively used in therapy for AMD. The photosensitive substance is not particularly limited, but porphrin derivatives presented in the section of Background art are preferred, and illustrative examples thereof include dendrimer-type porphyrin, verteporfin, SnET2, ATX-S10, MV6401, temoporfin, talaporfin and the like. In particular, dendrimer-type porphyrin is preferred.

When the polymer micelle is used as a carrier for the photosensitive substance, the photosensitive substance can be stabilized, and as is also clear from the Examples below, it can be efficaciously accumulated to CNV. Thus, efficacious occlusion of new vessels are enabled even at a low dose of laser. Therefore, less influence is exerted on ocular tissues, and continuous repetition of therapy for a long period of time is enabled, also leading to expectation to complete recovery of AMD.

Furthermore, many of photosensitive substances used in the PDT are slightly soluble in water; therefore, attempts have been made such as formation of a liposome preparation or the like to execute administration. However, in this instance, the preparation may be necessarily administered intravenously for a long period of time at regular intervals, thereby increasing burdens to the patients. When the polymer micelle is used, the photosensitive substance can be solubilized in water, therefore, administration by usual intravenous injection is enabled to eliminate the aforementioned burdens to the patients.

Furthermore, the PDT may also involve problems of light-sensitive disorder of skin. When the dendrimer-type porphyrin is used as the photosensitive substance, light-sensitive disorder is hardly found, suggesting an excellent characteristic of the dendrimer-type porphyrin.

### Brief Description of the Drawings

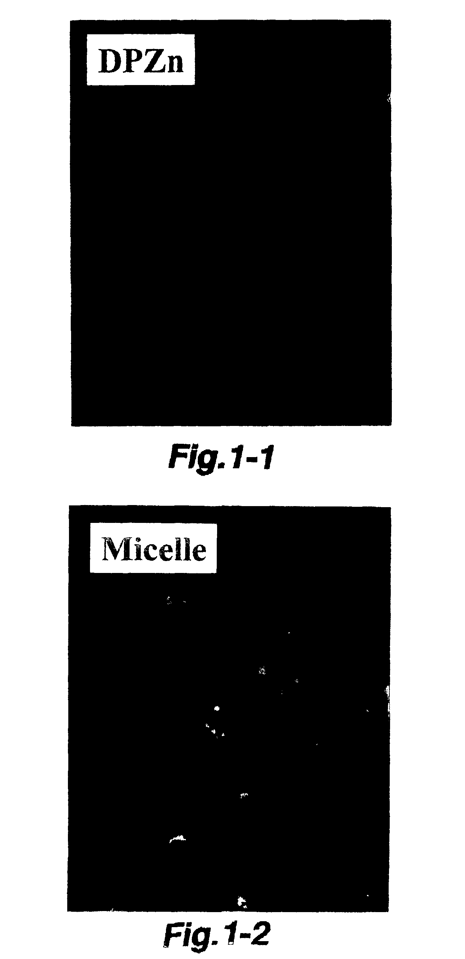

Fig. 1-1 is a fluorescence microgram obtained when DPZn and LLC cells were incubated.
Fig. 1-2 is a fluorescence microgram obtained when polymer micelles including DPZn and LLC cells were incubated.
Fig. 2-1 is a fluorescence microgram showing accumulating capability of polymer micelles incorporating DPZn to CNV examined 0.25 hour later.
Fig. 2-2 is a fluorescence microgram showing accumulating capability of polymer micelles incorporating DPZn to CNV examined 1 hour later.

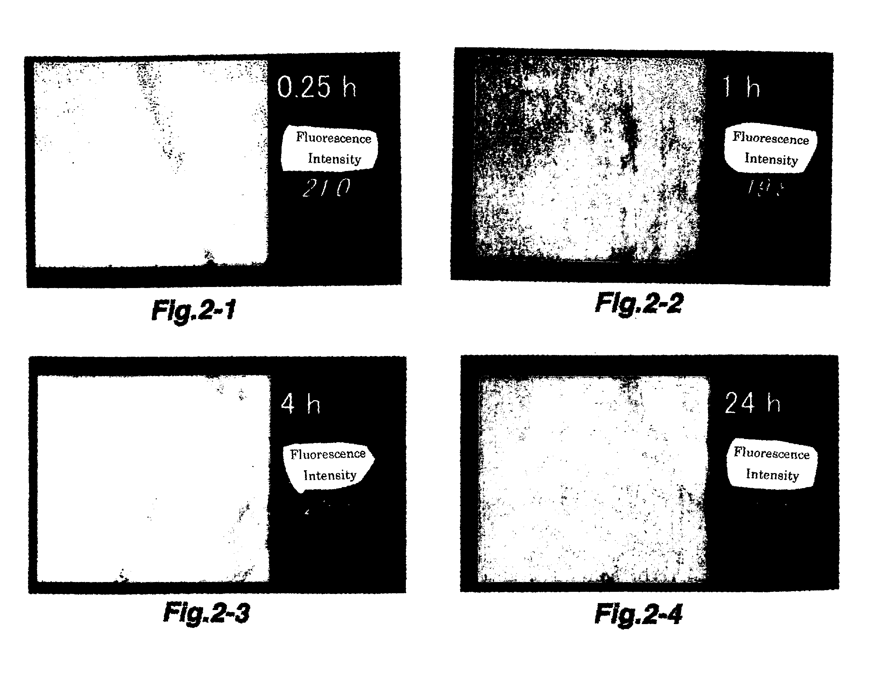

Fig. 2-3 is a fluorescence microgram showing accumulating capability of polymer micelles incorporating DPZn to CNV examined 4 hours later.
Fig. 2-4 is a fluorescence microgram showing accumulating capability of polymer micelles incorporating DPZn to CNV examined 24 hours later.

### Best Mode for Carrying Out the Invention

Next, some Examples of the present invention will be demonstrated.

### Example 1

### (Preparation of fluorescent-labelled polymer micelle)

A diblock copolymer having polyethylene glycol (PEG) as a hydrophilic polymer chain, and polyaspartic acid (P(Asp)) as an anionic polymer chain within one molecule was dispersed in water, and mixed with FITC-labelled polylysine (FITC-P(Lys)) to prepare a core-shell type PIC micelle solution (5 mg/mL) having a core of a polyion complex (PIC) consisting of P(Asp) and FITC-P(Lys), and a shell of PEG.

### (Production of choroidal neovascular (CNV) model)

After generally anesthetizing a BN rat by intramuscular administration of 1 mL/kg of a mixture of a 5% ketamine hydrochloride injection and a 2% xylazine hydrochloride injection (7:1), eye drops of a 0.5% tropicamide-0.5% phenylephrine hydrochloride ophthalmic solution were administered to render mydriasis. Then, photocoagulation was carried out with a semiconductor laser photocoagulator. Photocoagulation was carried out on six scattering positions per one eye in a posterior portion of the ocular fundus while focusing on retinal deep layer (coagulation conditions: spot size 75 µm, output 200 mW, coagulation time 0.05 sec) such that irradiation of large retinal vessels was avoided. After the photocoagulation, fundus photograph was taken to ascertain the site where the laser was irradiated.

### (Method of administration of polymer micelle)

PIC micelle in an amount of 400 µL was intravenously administered 7 days after the laser irradiation. As a control group, FITC, and FITC-P(Lys) were similarly administered, respectively, after dissolving in physiological saline to give 5 mg/mL.

### (Method of evaluation)

Following administration, eyeball was removed after a predetermined time, and a frozen tissue section was prepared. Thereafter, leakage of the fluorescence to the choroidal neovascular site was qualitatively observed by a fluorescence microscope.

### (Results)

In the PIC micelle administration group, accumulation of intense fluorescence was found agreeing with the choriocapillaris vascular lamina and choroidal new vessels one hour after the administration. Twenty four hours after the administration, fluorescence on the choroidal vascular lamina was attenuated, but accumulation of intense fluorescence agreeing with the choroidal new vessel remained. The fluorescence was also found 168 hours later.

In the FITC administration group, accumulation of intense fluorescence was found agreeing with the choriocapillaris vascular lamina and choroidal new vessel one hour after the administration, although no fluorescence was found 24 hours later.

In the FITC-P(Lys) administration group, the animal died one hour after the administration due to its toxicity.

### Example 2

Incorporation property into cells was examined using dendrimer-type porphyrin (DP) that is a photosensitive substance as a drug.

### (Preparation of polymer micelle incorporating DP)

Polymer micelle incorporating DP was prepared according to Example 1 described in Japanese Patent No. 3422481 (the same applied to in the following Examples).

DP used herein is an anionic porphyrin dendrimer [32(-)(L3)₄PZn] described in Example 1 in Japanese Patent No. 3422481 (hereinafter, referred to as DPZn).

### (Test on incorporation property into cells)

The polymer micelle incorporating DPZn (hereinafter, referred to as DPZn/polymer micelle), and LLC (Lewis Lung Carcinoma) cells were incubated in phosphate buffered saline in a dark place at 37°C for 8 hrs. After washing with a phosphate buffered saline, incorporation into the cells was qualitatively observed by a fluorescence microscope.

As a comparative control, DPZn and LLC cells were incubated under the same condition.

### (Results)

As shown in Fig. 1, much greater amount of the DPZn/polymer micelle was incorporated into the cells compared to DPZn, clearly showing the effect exerted by polymer micelle formation.

### Example 3

Using DPZn as a drug, accumulating capability to CNV was examined.

### (Method of administration)

The DPZn/polymer micelle was intravenously administered in a rat in which CNV was developed according to Example 1.

### (Method of evaluation and results)

Following the administration, eyeball was removed at a predetermined time. A frozen tissue section was prepared, and then accumulation to CNV was qualitatively observed by a fluorescence microscope. Consequently, as shown in Fig. 2, high accumulating capability was found agreeing with the CNV site at 0.25 hour, 1 hour, 4 hours and 24 hours after the administration.

### Example 4

Using DPZn as a drug, photosensitive erethism on the skin was examined.

### (Method of administration)

Abdominal hair of a rat in which CNV was developed according to Example 1 was shaved, and on the next day a DPZn/polymer micelle was intravenously administered.

As a comparative control, Photofrin was administered under the same condition.

### (Method of evaluation and results)

Four hours after the administration, a xenon lamp was irradiated on the shaved part (wavelength: 377-700 nm, power density: 30 mW/cm², irradiation area: 4 cm²). Time-dependent observation verified that photosensitive erethism was not found in the DPZn/polymer micelle administration group even after 2 weeks following irradiation, however, photosensitive erethism was found in the Photofrin administration group from one day after the irradiation.

### Example 5

Using DPZn as a drug, effect of CNV occlusion exerted by undergoing a PDT was examined.

### (Method of administration and undergoing PDT)

The DPZn/polymer micelle was dissolved in physiological saline (1.5 mg/ml; as DPZn concentration), and 400 µl of the solution was intravenously administered to a rat in which CNV was developed according to Example 1 (4 animals per 1 group). Laser for PDT was irradiated 0.25 hour and 4 hours after the administration (laser wavelength: 438 nm, power density: 600 mW/cm², spot size: 1120 µm). The irradiation dose was 5, 10, 25, 50, 100 J/cm².

On day 1 and 7 following the laser irradiation for PDT, fluorescein was intravenously administered, and leakage of fluorescein from CNV was observed with a fundus camera to evaluate the effect of CNV occlusion.

### (Results)

The results are presented in Tables 1 and 2. Proportion of CNV occlusion is shown by a mean value (4 animals per 1 group).

**Table 1:**

| PDT undergone 0.25 hour after DPZn/polymer micelle administration | | |
|---|---|---|
| Dose of laser (J/cm²) | Proportion of CNV occlusion (%) | |
| | Day 1 after undergoing PDT | Day 7 after undergoing PDT |
| 0 | 33.3 | 25.0 |
| 5 | 83.3 | 83.3 |
| 10 | 30.4 | 43.5 |
| 25 | 73.9 | 82.6 |
| 50 | 77.3 | 86.4 |
| 100 | 66.7 | 83.3 |

**Table 2:**

| PDT undergone 4 hours after DPZn/polymer micelle administration | | |
|---|---|---|
| Dose of laser (J/cm²) | Proportion of CNV occlusion (%) | |
| | Day 1 after undergoing PDT | Day 7 after undergoing PDT |
| 5 | 63.6 | 81.8 |
| 10 | 75.0 | 81.3 |
| 25 | 88.8 | 83.3 |
| 50 | 73.3 | 80.0 |
| 100 | 90.9 | 81.8 |

As is clear from the results shown in these Tables, sufficient effect of CNV occlusion was found even at a low dose of laser when porphyrin is incorporated into a polymer micelle. In addition, persistence of the effect was also elucidated.

Accordingly, it was revealed that the present invention is very useful in therapy for AMD.

### Industrial Applicability

The present invention relates to an ophthalmic drug delivery system characterized by efficiently delivering a drug to posterior tissues of eyeball such as choroid and retina, especially those wherein neovascularization has occured, by administering polymer micelles incorporating the drug that is useful as a therapeutic drug for an ocular disease. The drug delivery system of the invention can be effectively applied to photodynamic therapies when a photosensitive substance is used as the drug, and can be subjected to therapy for age-related macular degeneration through occluding choroidal new vessels.

## Claims

1. An ophthalmic drug delivery system comprising administering a polymer micelle incorporating a drug therein to deliver the drug to a posterior tissue of eyeball efficiently.

2. The drug delivery system according to claim 1 wherein the polymer micelle is formed with a block copolymer comprising a hydrophilic polymer chain as a shell and a hydrophobic polymer chain as a core.

3. The drug delivery system according to claim 2 wherein the hydrophilic polymer chain is polyoxyethylene or polyethylene glycol.

4. The drug delivery system according to claim 2 wherein the hydrophobic polymer chain is polylactone.

5. The drug delivery system according to claim 1 wherein the polymer micelle is formed with a block copolymer comprising a hydrophilic polymer chain as a shell and a charged polymer chain as a core.

6. The drug delivery system according to claim 5 wherein the charged polymer chain is a polyamine, a polycarboxylic acid or a polypeptide.

7. The drug delivery system according to claim 1 wherein the polymer micelle is a core-shell type polyion complex micelle comprising a hydrophilic polymer chain as a shell, and a charged polymer chain and a polymer electrolyte in a core.

8. The drug delivery system according to claim 7 wherein the charged polymer chain is an anionic polymer chain.

9. The drug delivery system according to claim 8 wherein the anionic polymer chain is polyaspartic acid.

10. The drug delivery system according to claim 7 wherein the charged polymer chain is a polyamine or a polycarboxylic acid.

11. The drug delivery system according to claim 7 wherein the polymer electrolyte is a polypeptide.

12. The drug delivery system according to claim 11 wherein the polymer electrolyte is polylysine.

13. The drug delivery system according to any one of claims 1 to 12 wherein the polymer micelle has a particle diameter of between 10 nm to 100 nm.

14. The drug delivery system according to any one of claims 1 to 12 wherein the administration method is intravenous injection.

15. The drug delivery system according to any one of claims 1 to 14 wherein the posterior tissue of eyeball is choroid or retina.

16. The drug delivery system according to claim 15 wherein new vessels are generated in the posterior tissue of eyeball.

17. The drug delivery system according to any one of claims 1 to 16 wherein the drug is a photosensitive substance.

18. The drug delivery system according to claim 17 wherein the photosensitive substance is used for a photodynamic therapy.

19. The drug delivery system according to claim 18 which is used for occlusion of choroidal new vessels.

20. The drug delivery system according to claim 18 which is used in a therapy for age-related macular degeneration.

21. The drug delivery system according to claim 18 wherein the photosensitive substance is a porphyrin derivative.

22. The drug delivery system according to claim 18 wherein the porphyrin derivative is a dendrimer-type porphyrin.

23. A therapeutic agent for age-related macular degeneration which comprises a photosensitive substance incorporated into a polymer micelle as an active ingredient, and which occludes choroidal new vessels by a photodynamic therapy.

24. The therapeutic agent for age-related macular degeneration according to claim 23 wherein the photosensitive substance is a porphyrin derivative.

25. The therapeutic agent for age-related macular degeneration according to claim 23 wherein the porphyrin derivative is a dendrimer-type porphyrin.
